# EUROPÄISCHE PATENTSCHRIFT

(11) **EP 1 390 746 B1**
(45) Veröffentlichungstag und Bekanntmachung des Hinweises auf die Patenterteilung: **27.09.2006**
(21) Anmeldenummer: 02732572.9
(22) Anmeldetag: 08.04.2002
(51) Int. Cl.: G01N 33/18, G01N 31/22, G01N 21/75

(54) **VERFAHREN ZUR BESTIMMUNG VON FLÜCHTIGEN SUBSTANZEN IN LÖSUNG**
METHOD FOR IDENTIFYING VOLATILE SUBSTANCES IN SOLUTION
PROCEDE POUR IDENTIFIER DES SUBSTANCES VOLATILES EN SOLUTION

(30) Priorität: 05.05.2001 DE 10121999
(43) Veröffentlichungstag der Anmeldung: 25.02.2004
(73) Patentinhaber: Merck Patent GmbH, 64293 Darmstadt (DE)
(72) Erfinder: KLIMANT, Ingo, 93098 Mintraching (DE); STANGELMAYER, Achim, 86633 Neuburg a.d. Donau (DE)
(86) Internationale Anmeldenummer: PCT/EP2002/003868
(87) Internationale Veröffentlichungsnummer: WO 2002/090975

(56) Entgegenhaltungen:
- EP-A- 0 663 239
- EP-A- 0 930 368
- CH-A- 649 383
- DE-A- 1 817 312
- US-A- 5 494 640
- US-A- 5 633 169
- LUEBBERS D W ET AL: "QUANTITATIVE FLUORESCENCE PHOTOMETRY WITH BIOLOGICAL FLUIDS AND GASES" ADVANCES IN EXPERIMENTAL MEDICINE AND BIOLOGY, SPRING ST., NY, US, Bd. 75, 1976, Seiten 65-68, XP000566843 ISSN: 0065-2598

## Beschreibung

Die Erfindung betrifft ein Verfahren zur photometrischen oder fluorimetrischen Bestimmung von flüchtigen Substanzen in Lösung.

Für bestimmte analytische Fragestellungen werden einzelne Komponenten durch Überführung in die Gasphase von dem Probengemisch abgetrennt. Diese gasförmigen Komponenten können dann mittels Gaschromatographie, Atomabsorption, IR- oder Chemolumineszenz-Spektroskopie bzw. mittels indirekter Methoden, wie Konduktometrie, Coulometrie, Potentiometrie, Gasvolumetrie, acidimetrischer Maßanalyse, manometrischer Messung, iodometrischer Maßanalyse, Fluorimetrie oder Photometrie, nachgewiesen werden.
Für bestimmte Fragestellungen wurden spezielle Verfahren entwickelt, wie beispielsweise in US 4 201 548. Dort wird ein Verfahren zur Bestimmung flüchtiger Substanzen in Flüssigkeiten beschrieben, wobei die Probe in ein mit einer semipermeablem Membran begrenztes Plastikgefäß gegeben wird. Nach Durchtritt durch die Membran färbt der Analyt eine mit Indikator imprägnierte Trägermembran. Diese Vorrichtung zur visuellen Detektion eignet sich jedoch nicht zur photometrischen oder fluorimetrischen Detektion.
US 5 275 956 beschreibt die Bestimmung von organischen Chlor-haltigen Verbindungen durch Chemolumineszenz. Die verwendete Vorrichtung enthält eine Aufschlusszone (Oxidationszone), eine Reaktionszone, in der die chemolumineszierende Verbindung entsteht, sowie eine Detektionszone. Weiterhin ist die sehr aufwendige Apparatur mit Verbindungsstücken, Pumpen und Detektoren versehen.

Daraus ergibt sich zunächst die Aufgabe, ein möglichst einfaches, universell einsetzbares Testsystem zu entwickeln, das es erlaubt, durch photometrische oder fluorimetrische Bestimmungen, unterschiedliche Stoffe aus verschiedensten Proben nachzuweisen.

Ein erster Ansatz hierzu wurde in EP 0 663 239 entwickelt. Es wird ein Testkit zur chemischen Analyse von gasförmigen Probeninhaltsstoffen beschrieben. Der Kit enthält eine Vorrichtung aus zwei einzelnen Behältern. Ein Behälter dient zur Aufnahme der Probe und Freisetzung des Gases, der andere zur Detektion. Das Gas wird von dem ersten Behälter über einen Adapter in den zweiten Behälter überführt. Nachteil des Kits ist die Notwendigkeit, zwei Behälter verwenden und diese während der Benutzung über einen Adapter verbinden zu müssen. Dies erfordert einen hohen experimentellen Aufwand. Zudem besteht bei zu langsamem Verbinden der beiden Behälter die Gefahr des Austritts bzw. Eintritts von Gas. Dadurch können die Analysedaten leicht verfälscht werden.

Aufgabe der vorliegenden Erfindung ist es daher, ein Verfahren zur fluorimetrischen oder photometrischen Bestimmung bereitzustellen, welches ermöglicht:
● ein breites Spektrum verschiedenster Proben auf unterschiedliche gasförmige oder in die Gasphase überführbare Stoffe hin zu untersuchen,
● das Verfahren mit geringem apparativem und experimentellem Aufwand durchzuführen,
● eine sehr selektive und empfindliche Analyse durchzuführen.

Es wurde gefunden, dass sowohl Selektivität und Empfindlichkeit als auch der experimentelle Aufwand der Bestimmung flüchtiger Substanzen in Proben stark verbessert werden kann, wenn Aufschluß und Detektion in einem Gefäß erfolgen.
Das Gefäß, beispielsweise eine Küvette, wird dazu durch eine gaspermeable Membran in mindestens zwei Reaktionsbereiche unterteilt.

Nach Zugabe der Probensubstanz und weiterer notwendiger Reagenzien wird das Gefäß verschlossen. Alle weiteren Analysenschritte wie Aufschluß, Reinigung und Detektion können in dem verschlossenen Gefäß durchgeführt werden.

In einer bevorzugten Ausführungsform besteht zumindest eine Membran, bevorzugt alle Membranen der erfindungsgemäßen Küvetten aus einem Siliconpolymer.

In einer weiteren bevorzugten Ausführungsform ist zumindest eine Membran, bevorzugt alle Membranen in die Küvette eingeklemmt.

In einer anderen bevorzugten Ausführungsform ist zumindest eine Membran, bevorzugt alle Membranen in die Küvette einpolymerisiert.

Gegenstand der Erfindung ist ein Verfahren nach Anspruch 1 zur Bestimmung von flüchtigen Substanzen in Proben unter Verwendung einer durch eine Membran in zwei Bereiche aufgeteilten Küvette, wobei
a) die Küvette geöffnet wird,
b) im oberen Teilbereich mit der Proben- und optionalen Zusatzreagenzien befüllt wird,
c) mit dem Verschluss abgedichtet wird,
d) die Küvette umgedreht wird,
e) die Probe aufgeschlossen wird, wobei der Aufschluss je nach Art er Probe mit oder ohne Aufschlusslösung erfolgen kann,
f) die Küvette erneut umgedreht wird,
g) die Indikatorlösung vermessen wird.

In Abbildung 1 ist eine geeignete Küvette dargestellt.
Abbildung 2 zeigt ein Analysengefäß mit einem zusätzlichen Teilbereich für Waschlösungen.
In Abbildung 3 ist ein Schema des erfindungsgemäßen Verfahrens zur Bestimmung von flüchtigen Substanzen in Lösung dargestellt.

Abbildung 4 zeigt ein Formteil mit integrierter gaspermeabler, ionenimpermeabler Membran, das sich durch seine Formgebung in das Gefäß eingespreizt werden kann.

Das erfindungsgemäße Verfahren eignet sich zum Nachweis verschiedenster Analyten, wie beispielsweise gasförmiger Verbindungen, die ohne oder mit vorheriger Umwandlung nachgewiesen werden können, flüchtiger organischer Verbindungen, wie Phenole oder halogenierte Kohlenwasserstoffe, oder Verbindungen, die durch eine in der Aufschlussteilküvette vorgeschaltete chemische Reaktion unter Gasentwicklung nachgewiesen werden, wie Nitrat, das zu NOₓ umgesetzt wird.
Beispiele sind in der nachfolgenden Tabelle gegeben:

| **Nachzuweisende Substanz** | **Analyt** |
|---|---|
| Total Organic Carbon (TOC) | CO₂ |
| Total Inorganic Carbon (TIC) | CO₂ |
| NH₄⁺ | NH₃ |
| CN- | HCN |
| Hypochlorid | Cl₂ |
| S²⁻ | H₂S |
| NO₃⁻ | NOₓ |
| HSO₃⁻ | SO₂ |
| CKW | |
| Flüchtige Amine | |
| Nicht-flüchtige quartäre Ammoniumionen (via Hoffmeister-Eliminierung) | |
| Tetramethylquecksilber | |
| Schwermetalle (indirekt) | |

Das erfindungsgemäße Verfahren beruht auf der Verwendung eines Gefäßes, in das zum einen die Indikatorlösung eingebracht werden kann und zum anderen die Probenlösung mit dem Aufschlussreagenz. Für eine Trennung der beiden Bereiche sorgt eine Membran.

Dazu wird zunächst die Indikatorlösung in das Gefäß gefüllt und anschließend die Membran eingebracht. Dann kann die Membran optional mit der Aufschlußlösung überschichtet werden. Nach Zugabe der Probe wird das Gefäß verschlossen.
Somit befinden sich alle zur Analyse benötigten Reagenzien in einem Gefäß. Dieses Konzept ermöglicht eine einfache und benutzerfreundliche Analyse der Proben. Die Arbeitsanleitung zur Analyse beschränkt sich auf folgende Schritte:
1. Aufschrauben des Gefäßes
2. Probe einpipettieren
3. Zuschrauben
4. Umdrehen
5. Aufschließen
6. Umdrehen
7. Vermessen

Das Gefäß oder die Analysevorrichtung ist bevorzugt aus Glas, oder aus einem anderen Material, z.B. einem organischen Polymer, bevorzugt optisch transparent oder zumindest versehen mit einem Fenster aus optisch transparenten Material. Bevorzugt besteht das Gefäß oder die Analysevorrichtung aus Materialien, die zur Herstellung von Photometerküvetten verwendet werden, wie z.B. Polycarbonat und besonders bevorzugt Quarzglas

Da das Gefäß während der Analyse umgedreht wird, enthält es einen flüssigkeits- und gasdichten Verschluss in Form eines Schraubdeckels oder Stopfens. In seiner bevorzugten Ausführungsform ist das Gefäß derart gestaltet, dass es für den Aufschluss der Probe beispielsweise in einen marktüblichen Trockenthermostaten und zur Detektion in ein übliches Messgerät als Messvorlage eingesetzt werden kann. Für photometrische oder fluorimetrische Analysetechniken ist deshalb die Ausgestaltung als Küvette bevorzugt. Diese Küvette kann rund, quadratisch oder rechteckig geformt sein. Im weiteren wird das erfindungsgemäße Gefäß als Küvette bezeichnet.

Unter den Begriff photometrische oder fluorimetrische Bestimmung fällt erfindungsgemäß auch die Trübungsmessung, z.B. Nephelometrie.

Zur Abtrennung des Aufschlussbereichs vom Detektionsbereich (Bereich der Indikatorlösung) wird in die Küvette eine gaspermeable und ionenimpermeable Membran eingebracht. Diese Membran ist impermeabel für Flüssigkeiten oder Ionen, insbesondere Protonen, und permeabel für Gase. Sie bildet eine an der Küvettenwand fest anliegende mechanisch stabile Abtrennung aus.

Die Membran weist bevorzugt die folgenden zusätzlichen Merkmale auf:
- Temperaturstabilität
- mechanische Stabilität (Druck, Zug)
- gleichmäßige Schichtdicke
- gute Haftung an der Wand der Küvette
- Zersetzungsstabilität gegenüber starken Basen.
Weiterhin sollte die Membran frei sein von kohlenstoffhaltigen Abspaltungsprodukten, die die Analyse von Kohlenstoff verfälschen oder sonstigen Abspaltungsprodukten, die die Indikatorlösung beeinflussen.

Diese Anforderungen werden von hydrophoben porösen, gaspermeablen Membranen, besonders gut von Siliconmembranen, Teflonmembranen und Kombinationsmembranen aus Silicon- und Teflonmembranen erfüllt.

Das Einbringen der Membran in die Küvette kann auf verschiedene Weise erfolgen. Sie kann beispielsweise durch Einkleben (z.B. direkt oder in einem Spalt oder auf einem Vorsprung), Einklemmen (z.B. direkt oder durch Einschub eines Formteils) oder Einpolymerisieren an der Innenwand der Küvette befestigt werden.
Im folgenden werden bevorzugte Möglichkeiten zum Einbringen der Membran näher erläutert:

### Einpolymerisieren der Membran:

Zum Einpolymerisieren sollten die Membran bzw. die Membranbestandteile auch die folgenden Eigenschaften aufweisen:
- Schwimmfähigkeit auf wässrigen Lösungen
- Polymerisationsfähigkeit auf einer stark basisch gepufferten Indikatorlösung
Besonders geeignet sind Membranen, die sich in relativ kurzen Polymerisationszeiten herstellen lassen.

Zunächst wird typischerweise die Indikatorlösung in das-Gefäß eingebracht. Dann wird eine Siliconmembran durch Auftropfen eines Siliconpräpolymers auf die Indikatorlösung erzeugt. Das flüssige Siliconpräpolymer schwimmt auf der Indikatorlösung und bildet einen gleichmäßigen Film aus. Nach der Polymerisation bildet der Siliconfilm eine hydrophobe, gas-permeable, ionen-impermeable fest mit der Gefäßwand verbundene Membran.

Bei der Herstellung von Siliconmembranen ist zwischen zwei Arten von Ausgangsstoffen zu unterscheiden: kondensationspolymerisierenden Silicone und additionsvernetzende Siliconen.
Während kondensationspolymerisierende Silicone sich durch eine leichte Verarbeitbarkeit der Präpolymere und eine gute Haftung an der-Glaswand des Gefäßes auszeichnen, ist ihr Einsatz für manche Anwendungen nicht möglich, da sie oft Rückstände von Lösungsmitteln oder kohlenstoffhaltigen Abspaltungsprodukten enthalten. Aus diesem Grund werden für die erfindungsgemäße Vorrichtung bevorzugt additionsvernetzende Silicone verwendet.

Anders als die kondensationsvernetzenden Silicone können diese nur begrenzt mit den Hydroxylgruppen der Glaswand reagieren. Deshalb wird bevorzugterweise die Glaswand zuvor teilweise oder vollständig hydrophobisiert. Diese Grundierung der Glaswand verhindert die Ausbildung eines stark gekrümmten Meniskus der flüssigkeitsoberfläche und garantiert so eine ausreichend große Kontaktfläche des Silicons mit der Glaswand. Die Hydrophobisierung der Glaswand erfolgt durch kovalente Kopplung von beispielsweise Trimethoxysilan (T9895, ABCR GmbH&Co) oder Propyltrimethoxysilan oder durch Beschichten der Glaswand mit einer dünnen Siliconschicht.
Bevorzugt ist auch die Verwendung von Glasgefäßen mit hydrophober Innenwand.

Ausgangsprodukte zur Herstellung von Siliconmembranen sind dem Fachmann bekannt. Typischerweise wird ein Copolymer aus einer linearen kettenförmigen Komponente (Base) mit reaktiven Endgruppen, und einer verzweigten Komponente (Vernetzer) mit reaktiven Endgruppen hergestellt. Als Reaktionsstarter für die chemische Reaktion, die zur Vernetzung führt, dienen, je nach Art der Base und des Vernetzers, verschiedene Chemikalien oder auch physikalische Parameter (Druckänderung, Temperaturänderung). Bei kondensationsvernetzenden Siliconpräpolymeren ist dies zumeist Wasser in Form des Wasserdampfanteils in der Luft, bei additionsvernetzenden Siliconpräpolymeren zumeist eine Chemikalie, ein sogenannter Radikalstarter.

### Einkleben der Membran:

Eine weitere bevorzugte Ausführungsform ist das Einkleben einer vorgefertigten Membran oder eines mit einer Membran beschichteten Membranträgers in das Gefäß.

Beispielsweise können vorgefertigte Membranen aus gaspermeablen Kunststoffen, bevorzugt Teflon oder anderen gaspermeablen Materialien, mittels eines Klebeschrittes mit der Küvettenwand verbunden werden.
Die verwendeten Klebstoffe sollten keine störenden Substanzen enthalten und in den Lösungsmitteln der eingesetzten Lösungen stabil sein.

Die verwendeten Klebstoffe sollten keine die Nachweisreaktion störenden Substanzen wie z.B. Lösungsmittel (Aceton, Toluol, Ethanol) enthalten, die während der Herstellung und Verarbeitung des Klebstoffes, oder während der späteren Aufschlussreaktion kohlenstoffhaltige Bestandteile freisetzen, und so die Nachweisreaktion beeinflussen. Weiterhin sollen die Klebstoffe in den Lösungsmitteln der eingesetzten Lösungen stabil sein.
Ein geeigneter Klebstoff für das Einkleben von Silikommembranen in die Küvette ist beispielsweise ein zweikomponenten additionsvernetzendes wasserstoffabspaltendes Silicon, wie z.B. Kwik-Sil ®, Kit silikon low viscosity, Vetrieb über World Precision Instruments, Inc

### Einklemmen der Membran:

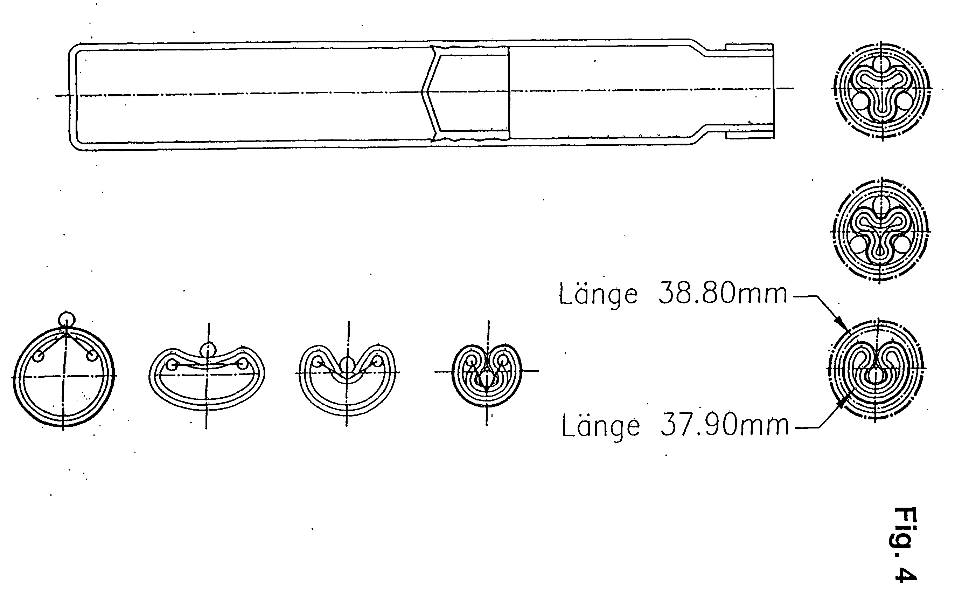

Eine weitere bevorzugte Ausführungsform ist das Einbringen eines Formteiles mit integrierter gaspermeabler, ionenimpermeabler Membran, das sich durch seine Formgebung in das Gefäß einspreizt. Das Formteil kann z.B. aus Silicon bestehen. Das Formteil ist bevorzugt technisch derart konstruiert, dass es, ähnlich einem zusammengedrückten Gummiball, selbsttätig aufspringt und sich dadurch in das Gefäß einspreizt. Ein derartiges Formteil in Abb. 4 beschrieben. Es ist das gefaltete Formteil (oben), das Entfalten des Formteils (unten rechts) und das in der Küvette befindliche entfaltete Formteil (unten links) gezeigt.
In einer bevorzugten Ausführungsform besteht die Membran zum Einklemmen in die Küvette aus einem Siliconpolymer, das faltbar ist. Auf diese Weise kann es in die Küvette eingebracht werden und entfaltet sich dann derart, dass es exakt und dicht in die Küvette eingepasst ist.

Demnach erfolgt die Herstellung einer erfindungsgemäßen Küvette mit eingeklemmter Trennmembran durch
- Bereitstellen einer geeigneten Küvette,
- Einfüllen der Indikatorlösung,
- Einschieben der gefalteten Membran mithilfe eines Werkzeugs,
- genaues Positionieren der Membran in der endgültigen Position oberhalb des Flüssigkeitsspiegels der Indikatorlösung,
- Lösen des Werkzeugs und damit Auffalten und Einspreizen der Membran in die Küvette
- optional Einfüllen einer Aufschlusslösung
- Verschließen der Küvette mit einer Verschlusskappe

Die Membran selbst kann in allen Ausführungsformen aus einer oder mehreren Komponenten bestehen.

Soll das Analysegefäß nur in zwei Teilbereiche unterteilt werden, kann nach dem Einbringen der Membran die Proben- bzw. Aufschlusslösung in den oberen Teilbereich gefüllt werden.

Falls bei einer Analyse störende flüchtige Komponenten neben dem Analyten zu erwarten sind, können zunächst Waschlösungen auf die Trennmembran gegeben werden und mit einer zweiten Membran überschichtet werden. Dadurch wird die Küvette in mehr als zwei, bevorzugt drei, Teilbereiche unterteilt. Diese Anordnung ist beispielsweise bei einem TOC Test in Anwesenheit von Schwefelwasserstoff sinnvoll. Zwischen Indikatorlösung und Proben- bzw. Aufschlusslösung ist ein weiterer Teilbereich durch eine zweite Membran abgeteilt, der Blei(II)-nitratlösung als Waschlösung enthält. Durch den Aufschluss treten sowohl CO₂ wie auch H₂S in die Gasphase über und durchdringen die erste Membran. Während das störende H₂S durch die Waschlösung zurückgehalten wird, gelangt das CO₂ durch die zweite Membran in die Indikatorlösung und erzielt dort einen Farbumschlag.

Unabhängig davon, auf welche Weise die Küvette unterteilt wird oder ob ein zusätzlicher Teilbereich mit Waschlösung in dem Gefäß enthalten ist, wird die Probenlösung und optional eine Aufschlusslösung in den obersten Teilbereich eingefüllt.

In diesem obersten Aufschluss-Teilbereich wird entweder die zu analysierende Substanz in eine flüchtige form überführt oder eine Reaktion durchgeführt, die eine flüchtige Substanz freisetzt, die als indirekter Nachweis für den Analyten dient. Dies kann mit oder ohne Zugabe einer besonderen Aufschlusslösung erfolgen. Während zum Nachweis von CO₂ oder NH₃ eine Aufschlußlösung verwendet wird, ist dies zum Nachweis beispielsweise flüchtiger Kohlenwasserstoffe nicht notwendig.
Mit Hilfe einer geeigneten Aufschlusslösung können selektiv bestimmte Stoffe in die Gasphase überführt werden. Beispielsweise können mit einem Puffer von pH 4 schwache Säuren bzw. ihre Gase ausgetrieben werden, starke Säuren jedoch nicht. Zusätzlich kann durch Erzeugung von Temperaturgradienten innerhalb der Küvette während des Aufschlusses, beispielsweise durch Aufheizen nur selektiv des Aufschlussbereichs, das entstehende Gas bevorzugt in den kälteren Indikatorteilbereich befördert werden.
Eine weitere Möglichkeit zur Anreicherung des Analyten im Indikatorteil besteht in der Verwendung unterschiedlich großer Volumina für Indikatorvolumen und Probenvolumen. Ist das Volumen der Probenlösung im Verhältnis größer als das der Indikatorlösung, so liegt das entstehende Gas in der Indikatorlösung in höherer Konzentration vor.

Obwohl die Detektion des Analyten mit der Indikatorlösung den letzten Schritt des erfindungsgemäßen Verfahrens darstellt, wird die Indikatorlösung ganz zu Anfang in das Analysengefäß gegeben. Erst nach 43efüllung mit der Indikatorlösung kann die erste Trennmembran auf die Lösung aufgebracht bzw. oberhalb der Lösung befestigt werden.

Die Indikatorlösung kann ein wäßriges bzw. ein organisches System oder eine Mischung aus beidem sein. Die Indikatoren können chromophore oder fluorophore Systeme sein. Genauso kann jedoch auch die Eigenfärbung oder Eigenfluoreszenz der Analyten detektiert werden. In diesem Fall dient die Indikatorlösung nur als Lösungsmittel für die gasförmige Komponente. Durch Variation des Indikators, des Puffervermögens der Lösung, des pH-Wertes oder der Zugabe von Komplexbildnern kann die Selektivität der Indikatorlösung entsprechend dem zu detektierenden Analyten variiert werden. Es können beispielsweise Farbumschlag, Fluoreszenzintensität oder Fluoreszenzlebenszeit detektiert werden.

Das erfindungsgemäße Analysengefäß eignet sich durch seinen einfachen Aufbau besonders zum Einsatz in Analysen-Kits. Auch in diesem Fall sollte die Form des Gefäßes passend für den direkten Einsatz in Thermostaten und Photometern oder sonstigen Analysegeräten sein. Daher ist die Verwendung einer Küvette bevorzugt.

Die im Analysen-Kit vorhandene Küvette ist mit der jeweiligen Indikatorlösung befüllt und der Indikatorteilbereich mit einer Membran gegenüber dem Aufschlussbereich abgegrenzt. Ist für die angestrebte Analyse eine weitere Aufreinigung des Analyten mittels Waschlösung notwendig, wird zunächst die Waschlösung auf die erste Membran gegeben und anschließend eine weitere Membran zur Abtrennung des Aufschlussbereichs aufgebracht. Ob die Aufschlusslösung bereits vor der Durchführung der Analyse in der Küvette gelagert wird, hängt von ihrer Stabilität ab. Im einen Fall wird die Aufschlusslösung bereits in die Küvette gegeben, so daß zur Durchführung der Analyse lediglich die Probenlösung zugegeben werden muß. Falls keine Aufschlusslösung notwendig ist oder die Ausschlusslösung aus Stabilitätsgründen separat gelagert wird, muss bei Durchführung der Analyse sowohl Probe als auch optional die Aufschlusslösung zugegeben werden. Nachdem die Küvette mit allen notwendigen Komponenten befüllt ist, wird sie flüssigkeits- und gasdicht verschlossen und dem erfindungsgemäßen Verfahren unterzogen.

Auf diese Weise ist es möglich, ohne großen experimentellen Aufwand, sehr selektive Analysen durchzuführen. Durch die optionale weitere Aufreinigung mittels einer Waschlösung können auch verunreinigte Realproben oder Proben mit störenden Komponenten vermessen werden. Eine Anreicherung oder Reinigung der Probe vor Einbringen in das Analysengefäß ist nicht notwendig.
Durch das geschlossene System können sehr empfindliche Messungen ohne Analytverlust durchgeführt werden.

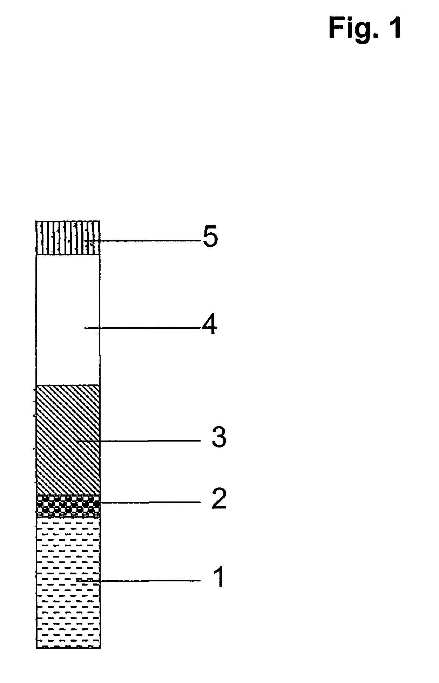

In Abbildung 1 ist ein geeignetes Gefäß dargestellt. Im Teilbereich (1) befindet sich die Indikatorlösung. Diese wird durch die semipermeable Membran (2) von der Aufschlusslösung im Teilbereich (3) getrennt. Bevorzugterweise nimmt die Aufschlusslösung nicht den ganzen Teilbereich (1) ein, sondern läßt einen Gasraum (4) frei. Der freie Gasraum in der Küvette ist für den Druckausgleich notwendig. Die Küvette wird mit einem Verschluss (5) abgedichtet.

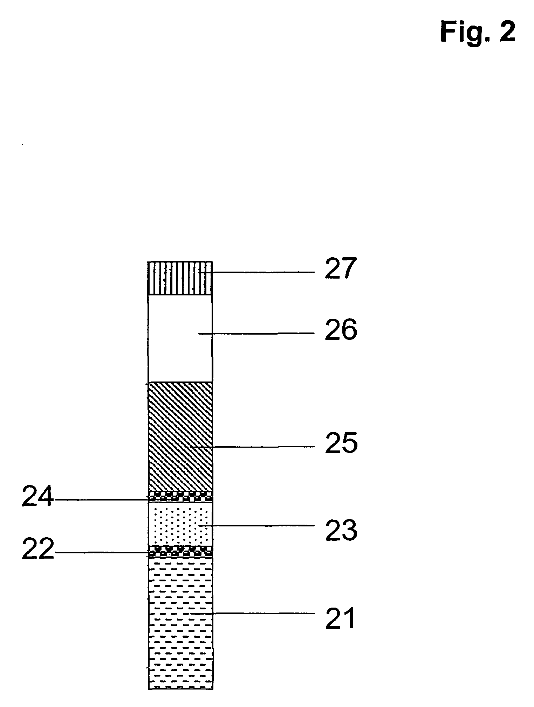

In Abbildung 2 wird die Indikatorlösung (21) von einer ionenimpermeablen gaspermeablen Membran (22) begrenzt. Es folgt eine Waschlösung (23), die von einer weiteren Membran (24) überschichtet ist. Oberhalb dieser Membran befindet sich die Aufschlusslösung (25) und der Gasraum (26). Die Küvette ist mit einem Verschluss (27) abgedichtet.

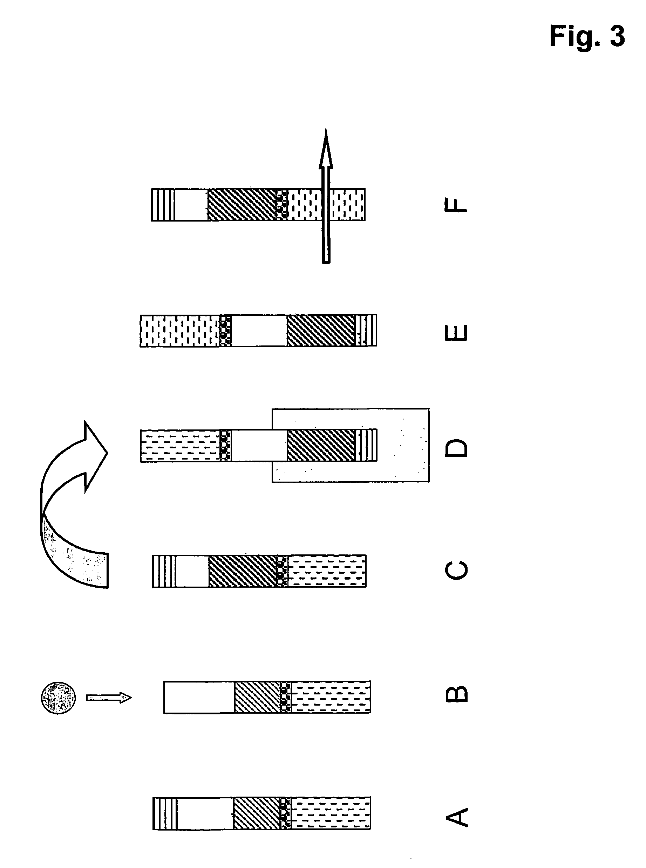

In Abbildung 3 ist ein Schema des erfindungsgemäßen Verfahrens zur Bestimmung von flüchtigen Substanzen in Lösung dargestellt. Die vorgefertigte, mit Indikator- und Aufschlusslösung befüllte Küvette (A) wird kurz geöffnet und die Probe zugegeben (B). Anschließend wird die Küvette erneut verschlossen (C), umgedreht und zum Aufschluss beispielsweise in einen Thermostaten eingeführt (D). Nach Beendigung des Aufschlusses (E) kann die Indikatorlösung nach erneutem Umdrehen des Gefäßes direkt in einem Photometer vermessen werden (F).

### Beispiele

### 1. Herstellung einer Küvette für die TOC-Bestimmung

### a) Hydrophobisierung

### Beispiel 1: Hydrophobisierung der Glaswand mit eine Siliconschicht.

Da additionsvernetzende Silicone keine gute Haftung an der Glaswand zeigen, wenn sie direkt auf die wässrige Pufferlösung gegeben werden, wird zunächst die Glaswand mit käuflichem Zweikomponentensilicon Kwik-Sil® der Firma World Precision Instruments Inc. hydrophobisiert.
Dazu werden die beiden Komponenten des Kwik-Sil® 1:1 Vol% mit Diethylether verdünnt, die beiden Lösungen vereint und die klare Flüssigkeit auf die Innenwand des Glasgefäßes in der Höhe aufgebracht, in der die Siliconmembran einpolymerisiert werden soll. Durch Drehbewegungen entlang der Längsachse wird ein gleichmäßiger dünner Ring aufgebracht. Nach 1 Minute ist der Ring trocken. Zur Entfernung eventueller Lösungsmittelreste wird die Küvette 2 Stunden bei 60 °C im Trockenschrank gelagert.

### Beispiel 2: Hydrophobierung der Glaswand mit einer kovalent gebundenen, kohlenstofffreien Silanschicht.

94 ml Ethanol, 1 ml Trimethoxysilan (T9895, ABCR GmbH&Co) und 4 ml Wasser werden gemischt. Der pH Wert wird mit verdünnte H₂SO₄ auf pH 4 bis 5 eingestellt. 0,3 ml dieser Lösung werden in ein aufrecht stehende Küvette einpipettiert. Die Küvette wird bei 140°C für eine Stunde in den Trockenschrank gestellt. Diese Gasphasensilanisierung führt zu einer effektiven Kopplung des Silans an die Glaswand und ergibt eine nahezu monomolekulare hydrophobe Beschichtung.

### b) Befüllen des Gefäßes mit der Indikatorlösung

4,5 ml einer Thymolblau Indikatorlösung (Pufferkonzentration Borat 3,5 mmol/l, pH 9,7, Thymolblau 0,06 mmol/l) werden in die Küvette gefüllt. Die Oberfläche der Indikatorlösung liegt dabei in Höhe der vorher einpolymerisierten Grundierung aus Silikon. Das Einfüllen der Indikatorlösung und das Aufbringen der Membran werden unter Stickstoff durchgeführt, um einen Eintrag von Kohlendioxid aus der Luft zu verringern. Durch die Hydrophobisierung der Glaswand bildet sich kein gekrümmter Meniskus der Wasseroberfläche aus, wodurch die Kontaktfläche Siliconmembran-Glaswand deutlich erhöht wird und die Siliconmembran eine einheitliche Schichtdicke erhält.

### c) Einpolymerisieren der Siliconmembran

2,5 ml Base (DMS-V22, Polydimethylsiloxane, vinyldimethyl terminated, Visc. 200 der Firma ABCR GmbH&Co, Karlsruhe) und 0,1 ml Vernetzer (HMS-301, Methylhydro-(65-70%) dimethylsiloxane copolymer der Firma ABCR GmbH&Co, Karlsruhe) werden gemischt und zu der klaren Lösung 2 µl Starter (PCO-075 der Firma ABCR GmbH&Co, Karlsruhe) unter starkem Rühren zugegeben. 0,5 ml der Siliconmischung werden auf die Oberfläche der Indikatorlösung getropft. Es bildet sich ein klare etwa 2 mm starke Siliconmembran aus. Diese Membran ist nach 10 Minuten bei Raumtemperatur fest. Zur Vervollständigung der Polymerisation wird die Küvette noch 1 Stunde unter Stickstoff gehalten und dann verschlossen.

### 2. Erfindungsgemäße Analyse von TOC

Ein TOC Testküvette ist aufgebaut aus folgenden Komponenten:
Zweikomponentensilicon für die Hydrophobisierung der Glaswand Name des Silikons: Kwik-Sil® ,
Kit silikon low viscosity, Vetrieb über World Precision Instruments,lnc Zweikomponentensilicon für die Membran DMS-V22, Polydimethylsiloxane, vinyldimethyl terminated, Visc. 200, base HMS-301, Methylhydro-(65-70%) dimethylsiloxane copolymer, crosslinker PCO-075, Platinium-divinyltetramethyldisiloxane complex, 3% Pt, catalyst Vetrieb über ABCR GmbH&Co.

### Hydrophobierung der Glaswand

Jede einzelne Komponente des Silikon Kwik-Sil® wird 1 zu 1 Vol% mit Diethylether verdünnt. Die beiden Lösungen werden vereint und die klare flüssige Mischung auf der Innenseite des Glasfläschchens in der Höhe aufgebracht, in der die Siliconmembran einpolymerisiert wird. Durch Drehbewegungen um die Längsachse des Glasfläschchens wird ein gleichmäßig dünner Ring aufgebracht. Das LM verdampft schnell und der dünne Film ist nach 1 min trocken. Die Fläschchen werden bei 60°C im Trockenschrank für 2 h gelagert, wobei alle Lösungmittelreste aus der sehr dünnen "Grundierung" ausgetrieben werden.

### Indikator

4,5 ml der blau gefärbten Indikatorlösung mit Pufferkonzentration Borat 3.5 mmol/l, pH Wert 9.7, Indikatorkonzentration Thymolblau 0.06 mmol/l werden in das Fläschchen eingefüllt. Die Oberfläche der Indikatorlösung liegt dabei in Höhe des vorher einpolymerisierten "Grundierung" aus Silikon. Das Einfüllen des Indikators und das Aufbringen der Silikonmembran werden unter Stickstoff durchgeführt, um einen Eintrag von Kohlendioxid aus der Luft zu verringern.

### Siliconmembran

2,5 ml Base (DMS-V22, Polydimethylsiloxane, vinyldimethyl terminated, Visc. 200) und 0.1 ml Vernetzer (HMS-301, Methylhydro-(65-70%) dimethylsiloxane copolymer) werden gemischt. Zu der klaren flüssigen Lösung werden 2µl Starter PCO-075 unter starkem Rühren zugegeben. Die Verarbeitungszeit nach Zugabe des Starters beträgt maximal 5 min. 0,5 ml der Siliconmischung werden auf die Oberfläche der Indikatorlösung getropft. Es bildet sich eine klare 2 mm starke Siliconmembran aus. Diese Membran ist nach 15 min. fest. Die Fläschchen werden zur Vervollständigung der Polymerisation stehend im Stickstoffstrom gelagert und nach 2 h mit einer Schraubkappe verschlossen.

### Versuchsdurchführung:

In jeweils drei der oben beschriebenen TOC Testküvetten werden 4,5 ml von Lösungen mit jeweils 25, 50, 100, 200, 400 mg/l Kohlenstoff einpipettiert. Die Küvetten werden mit einem Schraubverschluß verschlossen und auf dem Kopf stehen in einem Thermostaten (MERCK Thermoreaktor TR 200) mit 100°C gestellt. Nach 2 h Aufschlußzeit werden sie mit dem Photometer (MERCK SQ 118) vermessen.

## Patentansprüche

1. Verfahren zur Bestimmung von gasförmigen oder in die Gasform überführbaren Stoffen in Proben unter Verwendung einer Küvette, die durch eine ionen-impermeable, gaspermeable Membran (2) in zwei Bereiche aufgeteilt wird, in denen sich durch die Membran getrennt Raum für Proben- und Aufschlusslösung (3) und ein mit Indikatorlösung befüllter Raum für Indikatorlösung (1) befindet und die durch einen Verschluss (5) gas- und flüssigkeitsdicht verschlossen werden kann, **dadurch gekennzeichnet, dass**
a) die Küvette geöffnet wird,
b) im oberen Teilbereich mit der Proben- und optionalen Aufschlusslösungen befüllt wird,
c) mit dem Verschluss abgedichtet wird,
d) die Küvette umgedreht wird,
e) die Probe aufgeschlossen wird, wobei der Aufschluss je nach Art der Probe mit oder ohne Aufschlusslösung erfolgen kann,
f) die Küvette erneut umgedreht wird,
g) die Indikatorlösung vermessen wird.

2. Verfahren nach Anspruch 1, **dadurch gekennzeichnet, dass** eine Küvette eingesetzt wird, die durch mindestens zwei ionenimpermeable, gaspermeable Membranen (22, 24) in mindestens drei Bereiche aufgeteilt wird, in denen sich durch die Membranen getrennt Raum für Proben- und Aufschlusslösung (25), ein mit Indikatorlösung befüllter Raum für Indikatorlösung (21) und ein mit Waschlösung befüllter Raum für Waschlösungen (23) befindet.

3. Verfahren nach Anspruch 1 oder 2, **dadurch gekennzeichnet, dass** eine Küvette eingesetzt wird, in der zumindest eine ionenimpermeable, gaspermeable Membran aus einem Siliconpolymer besteht.

4. Verfahren nach einem oder mehreren der Ansprüche 1 bis 3, **dadurch gekennzeichnet, dass** eine Küvette eingesetzt wird, in die zumindest eine Membran eingeklemmt ist.

5. Verfahren nach einem oder mehreren der Ansprüche 1 bis 3, **dadurch gekennzeichnet, dass** eine Küvette eingesetzt wird, in die zumindest eine Membran einpolymerisiert ist.

6. Verwendung des Verfahrens nach einem oder mehreren der Ansprüche 1 bis 5 zur Bestimmung von TOC (total organic carbon).

## Claims

1. Method for the determination of gaseous substances or substances which can be converted into the gas form in samples using a cell which is divided by an ion-impermeable, gas-permeable membrane (2) into two zones in which are located, separated by the membrane, a compartment for sample and digestion solution (3) and a compartment for indicator solution (1) which is filled with indicator solution, and the cell can be sealed in a gas- and liquid-tight manner by a closure (5), **characterised in that**
a) the cell is opened,
b) filled with the sample and optional digestion solutions in the upper sub-zone,
c) sealed by means of the closure,
d) the cell is inverted,
e) the sample is digested, where the digestion can be carried out with or without digestion solution, depending on the nature of the sample,
f) the cell is re-inverted,
g) the indicator solution is measured.

2. Method according to Claim 1, **characterised in that** use is made of a cell which is divided by at least two ion-impermeable, gas-permeable membranes (22, 24) into at least three zones in which are located, separated by the membranes, a compartment for sample and digestion solution (25), a compartment for indicator solution (21) which is filled with indicator solution, and a compartment for washing solutions (23) which is filled with washing solution.

3. Method according to Claim 1 or 2, **characterised in that** use is made of a cell in which at least one ion-impermeable, gas-permeable membrane consists of a silicone polymer.

4. Method according to one or more of Claims 1 to 3, **characterised in that** use is made of a cell into which at least one membrane has been clamped.

5. Method according to one or more of Claims 1 to 3, **characterised in that** use is made of a cell into which at least one membrane has been polymerised.

6. Use of the method according to one or more of Claims 1 to 5 for the determination of TOC (total organic carbon).

## Revendications

1. Procédé pour la détermination de substances gazeuses ou de substances qui peuvent être converties selon la forme gazeuse dans des échantillons en utilisant une cellule qui est divisée par une membrane perméable aux gaz et imperméable aux ions (2) selon deux zones dans lesquelles sont localisés, en étant séparés par la membrane, un compartiment pour un échantillon et pour une solution de digestion (3) et un compartiment pour une solution d'indicateur (1) qui est rempli d'une solution d'indicateur, et la cellule peut être scellée d'une manière étanche aux gaz et aux liquides par une fermeture (5), **caractérisé en ce que**
a) la cellule est ouverte,
b) elle est remplie de l'échantillon et de solutions de digestion optionnelles dans la sous-zone supérieure,
c) elle est scellée au moyen de la fermeture,
d) la cellule est inversée,
e) l'échantillon est digéré, où la digestion peut être mise en oeuvre avec ou sans solution de digestion, en fonction de la nature de l'échantillon,
f) la cellule est re-inversée,
g) la solution d'indicateur est mesurée.

2. Procédé selon la revendication 1, **caractérisé en ce que** l'on utilise une cellule qui est divisée par au moins deux membranes perméables aux gaz et imperméables aux ions (22, 24) selon au moins trois zones dans lesquelles sont localisés, en étant séparés par les membranes, un compartiment pour un échantillon et une solution de digestion (25), un compartiment pour une solution d'indicateur (21) qui est rempli d'une solution d'indicateur, et un compartiment pour des solutions de lavage (23) qui est rempli d'une solution de lavage.

3. Procédé selon la revendication 1 ou 2, **caractérisé en ce que** l'on utilise une cellule dans laquelle au moins une membrane perméable aux gaz et imperméable aux ions est constituée en un polymère silicone.

4. Procédé selon une ou plusieurs des revendications 1 à 3, **caractérisé en ce que** l'on utilise une cellule dans laquelle au moins une membrane a été fixée.

5. Procédé selon une ou plusieurs des revendications 1 à 3, **caractérisé en ce que** l'on utilise une cellule dans laquelle au moins une membrane a été polymérisée.

6. Utilisation du procédé selon une ou plusieurs des revendications 1 à 5 pour la détermination de COT (carbone organique total).
